# EUROPEAN PATENT SPECIFICATION

(11) **EP 4 262 940 B1**
(45) Date of publication and mention of the grant of the patent: **27.11.2024**
(21) Application number: 21819234.2
(22) Date of filing: 01.12.2021
(51) Int. Cl.: A61M 15/00, A61M 15/06, A24F 42/60, A24F 42/00

(54) **INHALER ARTICLE AND MOUTHPIECE FOR USE WITH THE SAME**
INHALATORARTIKEL UND MUNDSTÜCK ZUR VERWENDUNG DAMIT
ARTICLE D'INHALATION ET EMBOUT BUCCAL À UTILISER AVEC CELUI-CI

(30) Priority: 16.12.2020 EP 20214434
(43) Date of publication of application: 25.10.2023
(73) Proprietor: Philip Morris Products S.A., 2000 Neuchâtel (CH)
(72) Inventor: CAMPITELLI, Gennaro, 2000 Neuchâtel (CH)
(74) Representative: Reddie & Grose LLP
(86) International application number: PCT/IB2021/061195
(87) International publication number: WO 2022/130090

(56) References cited:
- WO-A1-2018/037206
- WO-A1-2020/002924
- WO-A1-2020/148631
- WO-A1-2020/148633
- US-A1- 2020 214 360

## Description

The present disclosure relates to a mouthpiece for an inhaler article and to inhaler articles comprising the mouthpiece. Specifically, the present invention relates to a mouthpiece for powder inhalers.

Dry powder inhalers used to dispense powdered medicaments often strive to provide an entire dry powder dose in a single dose. Such dry powder inhalers are often complex in their design and may involve moving parts. In addition, these complex dry powder inhalers are difficult to produce and assemble at high speeds.

Dry powder inhalers that are constructed to provide dry powder particles to the lungs at inhalation (air flow) rates that are within conventional smoking regime inhalation (air flow) rates may be designed with linear airflow paths between the powder receptacle and the outlet. Dry powder inhalers may contain inhalable powder in a capsule that may be pierced to access the powder. However, loose powder may sometimes unintentionally fall out of such inhalers if the inhaler is inverted or otherwise manipulated in a non-upright position.

WO 2020/148631 A1 describes a dry powder inhaler having an air inlet, a reservoir and an airway assembly. The airway assembly has a first airway and a mouthpiece airway is connected to the first airway. An airway head is attached to the airway assembly, which extends into the reservoir cavity.

It is desirable to provide a mouthpiece for an inhaler article that mitigates unintentional powder leakage from the inhaler article. It is desirable to provide a mouthpiece for an inhaler article that captures loose powder within the inhaler in the event that the inhaler is turned upside down. It is desirable to provide a mouthpiece for an inhaler article that allows powder to be inhaled at inhalation (air flow) rates that are within conventional smoking regime inhalation (air flow) rates. It is desirable to provide a mouthpiece having a simple design that is easy to manufacture and an inhaler article that is easy to assemble.

All scientific and technical terms used herein have meanings commonly used in the art unless otherwise specified. The definitions provided herein are to facilitate understanding of certain terms used frequently herein and are not meant to limit the scope of the present disclosure.

The term "substantially" as used here has the same meaning as "significantly," and can be understood to modify the term that follows by at least about 90 %, at least about 95 %, or at least about 98 %.

The term "not substantially" as used here has the same meaning as "not significantly," and can be understood to have the inverse meaning of "substantially," i.e., modifying the term that follows by not more than 25 %, not more than 10 %, not more than 5 %, or not more than 2 %.

For the purpose of the present description and of the appended claims, except where otherwise indicated, all numbers expressing amounts, quantities, percentages, and so forth, are to be understood as being modified in all instances by the term "about." Also, all ranges include the maximum and minimum points disclosed and include any intermediate ranges therein, which may or may not be specifically enumerated herein. In this context, therefore, a number A is understood as A ± 5 % of A. Within this context, a number A may be considered to include numerical values that are within general standard error for the measurement of the property that the number A modifies. The number A, in some instances as used in the appended claims, may deviate by the percentages enumerated above provided that the amount by which A deviates does not materially affect the basic and novel characteristic(s) of the claimed invention. Also, all ranges include the maximum and minimum points disclosed and include any intermediate ranges therein, which may or may not be specifically enumerated herein.

Terms such as "a," "an," and "the" are not intended to refer to only a singular entity, but include the general class of which a specific example may be used for illustration.

The terms "a," "an," and "the" are used interchangeably with the term "at least one." The phrases "at least one of" and "comprises at least one of" followed by a list refers to any one of the items in the list and any combination of two or more items in the list.

As used here, the term "or" is generally employed in its usual sense including "and/or" unless the content clearly dictates otherwise. The term "and/or" means one or all of the listed elements or a combination of any two or more of the listed elements.

As used here, "have", "having", "include", "including", "comprise", "comprising" or the like are used in their open-ended sense, and generally mean "including, but not limited to." It will be understood that "consisting essentially of," "consisting of," and the like are subsumed in "comprising" and the like. As used herein, "consisting essentially of," as it relates to a composition, product, method or the like, means that the components of the composition, product, method or the like are limited to the enumerated components and any other components that do not materially affect the basic and novel characteristic(s) of the composition, product, method or the like.

The words "preferred" and "preferably" refer to embodiments that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure, including the claims.

In the present disclosure, an inhaler article is provided that is suitable for inhaling a dry powder. The inhaler article generally includes an inhaler body and a mouthpiece element coupled with the inhaler body. The inhaler body includes a receptacle for housing an inhalable powder. An airflow path is provided through the mouthpiece element.

In some embodiments, the inhaler article may be used with a holder. For example, the inhaler article may be configured for use with a holder that is capable of opening a closed distal end of the inhaler article. The inhaler article may be configured for use with a holder that is capable of piercing a dry powder capsule housed in the receptacle. The inhaler article may be configured for use with a holder that provides an air inlet for the inhaler article.

According to embodiments, the mouthpiece element mitigates unintentional powder leakage from the inhaler article. The mouthpiece element is constructed to reduce or prevent loose powder from falling out of the inhaler article in the event that the inhaler article is turned upside down. The mouthpiece element allows powder to be inhaled at inhalation (air flow) rates that are within conventional smoking regime inhalation (air flow) rates, such as 5 L/min or less, or 2 L/min or less. The mouthpiece element has a simple design that is easy to manufacture. The mouthpiece element and inhaler article are easy to assemble.

According to an aspect of the present invention, there is provided an inhaler article comprising an inhaler body comprising a tubular side wall defining an interior and a longitudinal center axis. The interior forms a receptacle for housing an inhalable powder. The inhaler article further comprises a mouthpiece element extending along the longitudinal center axis from a distal end to a proximal end. "Proximal" means the end to be accessed by the mouth of a user when in use. "Distal" means the end opposite the proximal end when in use. The distal end of the mouthpiece element is received in the interior of the inhaler body. The mouthpiece element comprises a tube extending coaxially with the inhaler body. The tube comprises an interior surface having a first diameter. The mouthpiece element comprises a blocker disposed at the distal end of the mouthpiece element, coaxial with the tube. The mouthpiece element comprises a collector surface. The mouthpiece element comprises an airflow channel extending through the mouthpiece element. The airflow channel comprises an off-set portion extending from the receptacle to the collector surface, and a discharge portion extending to an outlet at the proximal end of the mouthpiece element. The off-set portion is substantially parallel to and laterally off-set from the discharge portion.

According to an embodiment, the inhaler article of the present disclosure mitigates unintentional powder leakage from the inhaler article. The inhaler article includes one or more features that capture loose powder within the inhaler in the event that the inhaler is turned upside down. According to an embodiment, the inhaler article of the present disclosure comprises a mouthpiece with a blocker and a collector surface that are arranged such that the amount of powder inadvertently falling out of the inhaler is reduced or minimized when the inhaler is inverted. The collector surface is configured to collect or capture any loose powder inside the inhaler. The collector surface prevents or reduces the amount of powder falling out of the receptacle when the inhaler is inverted. According to an embodiment, the inhaler article of the present disclosure comprises a mouthpiece that has an off-set portion in the airflow channel and a collector surface where powder is collected. By providing a blocker and an airflow channel with an off-set portion (for example, a non-linear airflow channel), loose powder does not simply fall out of the receptacle through the airflow channel. Due to the design of the mouthpiece, any loose powder collected or captured on the collector surface may be dropped back into the receptacle by turning the inhaler upright. Alternatively, the inhaler article and mouthpiece may be cleaned by turning the inhaler article upside down and tapping the inhaler article against a surface (for example, a table). The inhaler article may be tapped several times to cause loose powder to fall out.

The inhaler article of the present disclosure allows powder to be inhaled at inhalation (air flow) rates that are within conventional smoking regime inhalation (air flow) rates. The inhaler body and the mouthpiece have a simple design that is easy to manufacture and easy to assemble into an inhaler article. According to an embodiment, the mouthpiece may be simply inserted into a tubular inhaler body.

According to an embodiment, the discharge portion of the mouthpiece airflow channel is parallel to the longitudinal center axis. The discharge portion may be coaxial with the longitudinal center axis. According to an embodiment, the off-set portion of the mouthpiece airflow channel is parallel to the longitudinal center axis. The off-set portion may be coaxial with the longitudinal center axis. In some embodiments, both the discharge portion and the off-set portion are parallel to or coaxial with the longitudinal center axis, while being laterally off-set from one another. According to an embodiment, the inhaler article does not include an airflow path extending linearly from the receptacle to the outlet. The airflow channel may comprise an intermediate portion connecting the off-set portion and the discharge portion. The intermediate portion may extend radially inward from the off-set portion.

The collector surface is a surface of the mouthpiece that is constructed and configured to stop and/or capture loose particles inside the inhaler article when the inhaler article is turned from an upright position. An upright position in this context is considered to be the position where the proximal end and the mouthpiece of the inhaler article are pointing upward. For example, when the inhaler article is turned 90 degrees or more (for example, from 135 to 180 degrees) from the upright position, the collector surface may capture loose particles that may fall from the receptacle toward the proximal end. The collector surface may form a ring. For example, the collector surface may form a ring around a portion of the airflow channel. According to an embodiment, the inner diameter of the collector ring is greater than the first diameter (that is, the inner diameter of the tube). The outer diameter of the collector ring is greater than the second diameter (that is, the diameter of the blocker).

According to an embodiment, the mouthpiece element comprises a blocker disposed at the distal end of the mouthpiece element. The blocker may be arranged to block airflow through the center of the mouthpiece element at the distal end of the mouthpiece element.

According to an embodiment, the tube defines an outlet at the proximal end of the mouthpiece element. The proximal end of the mouthpiece element may be the mouth end of the inhaler article. The distal end of the mouthpiece element is inserted into or disposed inside the inhaler body. In some embodiments, a portion of the mouthpiece element is disposed inside the inhaler body. A portion of the mouthpiece element (for example, the mouth end) may be outside of the inhaler body. In one embodiment, a majority of or the entire mouthpiece element is disposed inside the inhaler body. The distal end of the mouthpiece element may form a proximal end of the receptacle. The receptacle may extend from the distal end of the inhaler body to the distal end of the mouthpiece element. The inhaler body may have a closed distal end. The distal end of the inhaler body may have a folded closure. For example, the distal end of the inhaler body may be folded closed by a fan fold. The distal end of the inhaler body may form the distal end of the receptacle.

According to an embodiment, the receptacle is constructed to receive a capsule containing inhalable powder. In some embodiments, the receptacle comprises a capsule containing inhalable powder. The inhaler article may be suitable for inhalation and delivery of various different inhalable powders. According to an embodiment, the capsule contains dry powder comprising particles containing one or more pharmaceutically active agents. Examples of pharmaceutically active agents include nicotine, anatabine, antiviral compounds such as acyclovir; anti-inflammatory compounds such as salicylic acid, aceclofenac, or ketoprofen; antidiabetic compounds such as metformin or glipizide; antihypertensive compounds such as oxprenolol; antiemetic compounds such as promethazine; antidepressant compounds such as seproxetine; anticoagulant compounds such as picotamide; bronchodilators such as clenbuterol; or anticancer compounds such as beta-lapachone. The pharmaceutically active agent may include a pharmaceutically acceptable salt of the pharmaceutically active agent. Suitable salts include, for example, a salt of lactic acid ("lactate"), tartaric acid ("tartrate" or "bitartrate"), aspartic acid ("aspartate"), pyruvic acid ("pyruvate"), citric acid ("citrate"), salicylic acid ("salicylate"), glutamic acid ("glutamate"), gentisic acid ("gentisate"), benzoic acid ("benzoate"), fumaric acid ("fumarate"), hydrochloric acid ("hydrochlorate"), alfa-resorcylic acid ("alfa-resorcylate"), beta-resorcylic acid ("beta-resorcylate"), oxalic acid ("oxalate"), p-anisic acid ("anisate"), glutaric acid ("glutarate"), and the like. In some cases, the capsule contains nicotine powder. For example, the capsule may contain a dry powder comprising nicotine salt. The dry powder may further contain other components, such as sugar or sugar alcohol, amino acid, flavorant, cough suppressant, or other pharmaceutically acceptable ingredients that are suitable for use in inhalable powders. In one embodiment, the capsule contains nicotine powder comprising nicotine particles, where the nicotine particles comprise nicotine salt, sugar or sugar alcohol, and amino acid. The capsule may further comprise flavor particles, cough suppressant particles, or both flavor and cough suppressant particles. Flavor and cough suppressant particles are collectively referred to here as flavor particles. The capsule may contain an inhalable powder comprising pharmaceutically active agent particles with MMAD particle size in the range of 0.5 µm to 10 µm, or 0.5 µm to 5 µm. In one embodiment, the capsule contains nicotine powder comprising nicotine particles, where the nicotine particles have MMAD particle size in the range of 0.5 µm to 10 µm, or 0.5 µm to 5 µm. The capsule may further contain flavor particles having MMAD particle size of 10 µm or greater, 20 µm or greater, or 40 µm or greater. The flavor particles may have MMAD particle size of 200 µm or less, 150 µm or less, or 120 µm or less. The flavor particles may have MMAD particle size of 20 µm to 200 µm or from 40 µm to 120 µm. In some embodiments, the capsule contains nicotine particles having MMAD particle size in the range of 0.5 µm to 10 µm, or 0.5 µm to 5 µm and flavor particles having MMAD particle size of 20 µm to 200 µm or from 50 µm to 150 µm. In a preferred embodiment, the capsule contains nicotine powder.

The inhaler body and the mouthpiece element may be constructed of any suitable materials. In some embodiments, the mouthpiece element is constructed of injectable, extrudable, or moldable polymeric material. In a preferred embodiment, the mouthpiece element is constructed of injectable polymeric material. Any suitable polymer may be used. In a preferred embodiment, the mouthpiece element is made of injection molded bioplastic. A bioplastic may be a polymer that is made from biobased ingredients or is biodegradable, or that is both made from biobased ingredients and is biodegradable. Biobased ingredients may be evaluated based on European standard CEN/TS 16137:2011. Biodegradability may be determined based on European standard EN13432.

The inhaler body may be constructed of one or more of paper, paperboard, cardboard, reconstituted tobacco paper, and cellophane. In some embodiments, the inhaler body is made of cellulosic material, such as paper, any other paper-based material, any other cellulose-based material, or a bioplastic-based material. Preferably the inhaler body is made from a biodegradable material. In a preferred embodiment, the inhaler body is made of paper or cardboard. The paper-based material may be bleached or unbleached. Paper-based materials may be one or more of light, cheap, and biodegradable. Preferably, the inhaler body exhibits sufficient mechanical strength and stiffness to withstand significant deformation during interaction with the receptacle, including the insertion of the mouthpiece element into the inhaler body. The inhaler body may be formed from a paper sheet. The inhaler body may be cut from a continuous sheet of paper. The inhaler body may be rolled from a continuous sheet of paper. This may simplify manufacturing of the inhaler body and the mouthpiece element.

The inhaler body and the mouthpiece element may be formed from materials that are biodegradable. Most preferably, the inhaler body is formed from a paper-based material, such as paper, paperboard or cardboard, and the mouthpiece element is formed from bioplastic, such as injection molded bioplastic.

The mouthpiece element may be designed to exhibit a desired resistance to draw ("RTD"). The RTD of a specimen refers to the static pressure difference between the two ends of the specimen when it is traversed by an air flow under steady conditions in which the volumetric flow is 17.5 milliliters per second at the output end. The RTD of a specimen may be measured using the method set out in ISO Standard 6565:2002. The mouthpiece element may have an RTD ranging from 30 to 200 mmWG. The mouthpiece airflow channel may be constructed to have any suitable dimensions to achieve a desired RTD. For example, the discharge portion of the airflow channel may have a cross sectional area of 2 mm² to 15 mm² or from 3 mm² to 13 mm².

The inhaler article may further comprise a wrapper wrapped about at least a portion of the inhaler body. The wrapper may also be wrapped about at least a portion of the mouthpiece element. In some embodiments, the wrapper does not cover the entire length of the mouthpiece element such that a portion of the length of the mouthpiece element is left uncovered. The wrapper may be a paper wrapper, such as a cigarette wrapper or tipping paper. The wrapper may be adhered by an adhesive.

According to an embodiment, the distal end of the mouthpiece element is inserted into or disposed inside the inhaler body. In some cases, the entire mouthpiece element may be inserted into or disposed inside the inhaler body. In some embodiments, the mouthpiece element comprises a tubular side wall extending from the proximal end of the mouthpiece to the distal end of the mouthpiece. The mouthpiece element may comprise an inner tube that forms a portion of the airflow channel. The tubular side wall may circumscribe the inner tube. The tubular side wall may be substantially straight, extending linearly along the length of the mouthpiece element. The mouthpiece element may have an outer diameter that is slightly smaller than the inner diameter of the inhaler body such that the mouthpiece element may be completely disposed inside the inhaler body. The proximal end of the mouthpiece element may be aligned with a proximal end of the inhaler body.

The mouthpiece element may comprise a blocker that is in the shape of a cylinder. The cylindrical blocker may be coaxial with the longitudinal center axis of the mouthpiece element. The mouthpiece element may further comprise a plurality of brackets extending from the blocker to the side wall of the mouthpiece element. The plurality of brackets may be arranged to maintain the blocker in place inside the side wall. The plurality of brackets may be arranged symmetrically about the blocker.

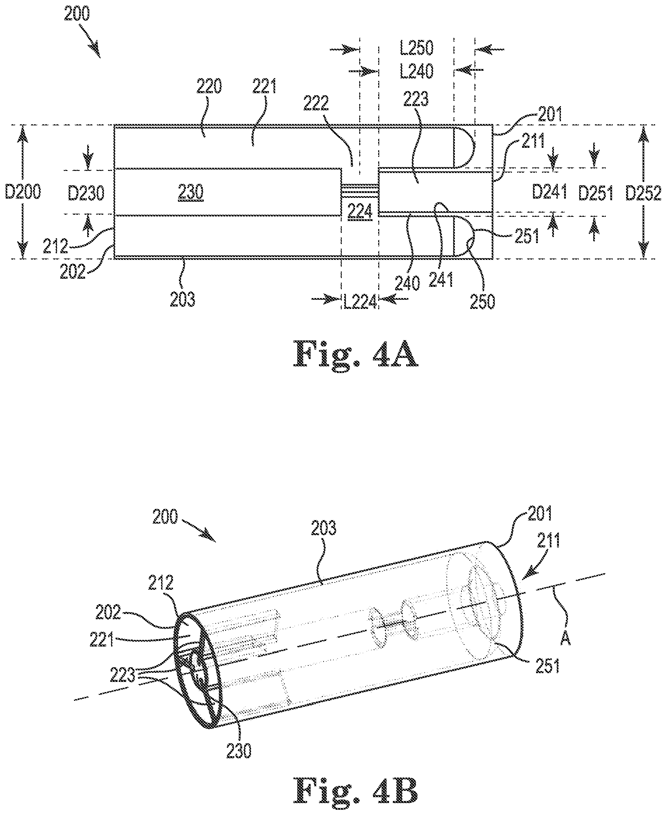

The inner tube may extend coaxially within the tubular side wall. The inner tube may form the proximal-most portion of the airflow channel. Thus, the discharge portion of the airflow channel may extend through the inner tube. The inner tube and therefore the discharge portion may extend along the longitudinal center axis. The blocker may be disposed at the distal end of the mouthpiece and may have a diameter that is equal to or greater than the inside diameter of the inner tube. The blocker and the inner tube may be coaxial. The blocker and the inner tube may be arranged such that a gap is left between the blocker and the inner tube. The gap may have an axial distance or length of 1 mm to 5 mm, or from 1.5 mm to 3 mm. The length of the gap (shown as L224 in FIGURE 4A) may be selected in view of the length between the axial midpoint of the gap and the proximal end of the collector surface (shown as L250 in FIGURE 4A). When powder is being inhaled, some of the powder may become entrained in the space between the inner tube and the tubular side wall of the mouthpiece element. If the distance from the gap to the collector surface is increased, the length of the gap may also be increased to maintain the amount or powder discharged from the inhaler. A ratio of the length of the gap to the length between the axial midpoint of the gap and the proximal end of the collector surface, L224:L250, may range from 2 to 4 or from 2.5 to 3.5. In a preferred embodiment the ratio is about 3.

The off-set portion of the airflow channel may be arranged around the blocker. The off-set portion of the airflow channel may be formed of a plurality of channels disposed about the blocker. The plurality of channels may be symmetrically disposed about the blocker. The blocker may be held in place by brackets extending from the blocker to the tubular side wall of the mouthpiece element.

The collector surface may form a ring surrounding the inner tube. In some cases, the collector surface extends from the inner tube to the tubular side wall of the mouthpiece element. The collector surface may have any suitable shape. Preferably, the collector surface is shaped to enable capture of loose particles and return of the collected or captured particles to the receptacle. In some embodiments, the collector surface comprises a curved cross sectional shape in a cross section taken along the longitudinal center axis of the mouthpiece element. Preferably, the curved cross sectional shape is concave toward the distal end of the mouthpiece element.

The collector surface may be disposed proximally of the blocker. The collector surface may be disposed proximally of the gap between the blocker and the inner tube. At least a portion of the inner tube may extend from the collector surface toward the distal end of the mouthpiece element. The portion of the inner tube extending distally from the collector surface may have a length of 2 mm to 6 mm.

The mouthpiece airflow channel may be constructed to have any suitable dimensions to achieve a desired RTD. At the distal end, the available open area for airflow is limited by the size of the blocker. The inner diameter of the tubular side wall may be about 5 mm to about 8 mm. The blocker may have a diameter of 1.5 mm to 4 mm, or from 2 mm to 3 mm. At the proximal end, the available area for airflow is determined by the size of the discharge portion. The discharge portion of the airflow channel may have a cross sectional area of 10 mm² to 20 mm² or from 11 mm² to 14 mm². The mouthpiece element may exhibit an RTD of 30 mmWG or greater, 35 mmWG or greater, or 40 mmWG or greater. The RTD may be 75 mmWG or less, 60 mmWG or less, or 50 mmWG or less. For example, the RTD may be from 30 to 75 mmWG, preferably from 35 to 60 mmWG, more preferably from 40 to 50 mmWG. In one embodiment, the RTD is about 45 mmWG.

According to an embodiment, the distal end of the mouthpiece element is inserted into or disposed inside the inhaler body. In some cases, only a distal portion of the mouthpiece element is inserted into or disposed inside the inhaler body. That is, the mouthpiece element is only partially inserted inside the inhaler body. In some embodiments, the mouthpiece element comprises a recessed portion having a cross-sectional dimension (for example, diameter) that is smaller than the cross-sectional dimension (for example, diameter) of the blocker. The distal end portion of the mouthpiece element may be the blocker. The recessed portion may comprise a groove circumscribing the mouthpiece element. The recessed portion may define the collector surface. The recessed portion may comprise a plurality of airflow channel openings. The mouthpiece element may be inserted into the inhaler body such that at least the blocker and the recessed portion are disposed inside the inhaler body.

In some embodiments, the off-set portion of the airflow channel is formed between the tubular side wall of the inhaler body and a distal end portion of the mouthpiece element. The off-set portion of the airflow channel may extend from the receptacle to the recessed portion. The distal end portion of the mouthpiece element (for example, the blocker) may be disposed inside the inhaler body. The off-set portion of the airflow channel may be formed between the tubular side wall of the inhaler body and the blocker.

The airflow channel may further comprise an intermediate portion comprising at least one intermediate channel extending from at least one airflow channel opening to the discharge portion. The airflow channel may comprise a plurality of intermediate channels extending from the plurality of airflow channel openings to the discharge portion. The airflow channel may comprise one intermediate channel. The airflow channel may comprise two intermediate channels. The airflow channel may comprise three intermediate channels. The airflow channel may comprise four intermediate channels. In a preferred embodiment, the airflow channel comprises four intermediate channels. The plurality of intermediate channels are in fluid communication with the off-set portion and the discharge portion, connecting the two portions of the airflow channel. The plurality of intermediate channels may extend radially inward from the recessed portion. The plurality of channels may be orthogonal to the tube. The plurality of intermediate channels may be orthogonal to the discharge portion. The plurality of intermediate channels may be distributed symmetrically about the longitudinal center axis.

The mouthpiece element may comprise a molded shape. Preferably, the mouthpiece element comprises a cylindrical tube. The recessed portion may comprise a groove circumscribing the cylindrical tube. The mouthpiece element may comprise multiple sections or portions having varying cross sectional dimensions (for example, diameters). For example, the mouthpiece element may comprise a first reduced portion separating the mouth end disposed at the proximal end of the mouthpiece element from a main portion of the mouthpiece element. The first reduced portion may have a cross sectional dimension (for example, diameter) that is smaller than the cross sectional dimension (for example, diameter) of the mouth end and the cross sectional dimension (for example, diameter) of the main portion. The mouthpiece element may further comprise a second reduced portion between the main portion of the mouthpiece element and the recessed portion. The second reduced portion may have a cross sectional dimension that is smaller than a cross sectional dimension of the main portion and greater than the cross sectional dimension of the recessed portion. The difference in the dimensions of the second reduced portion and the main portion may be only slight. The difference in the dimensions of the second reduced portion and the main portion may account for the thickness of the tubular wall of the inhaler body such that when assembled, the inhaler wall appears to continue at about the same circumference from the inhaler body to the mouthpiece element. The difference in the dimensions of the second reduced portion and the main portion may be equal to the thickness of the tubular wall of the inhaler body.

The inhaler article may further comprise a wrapper. The transition from the inhaler body to the mouthpiece element may be covered by the wrapper. The wrapper may be wrapped about a portion of the mouthpiece element and at least a portion of the inhaler body. For example, the wrapper may extend distally from the main portion of the mouthpiece element. The first reduced portion and the mouth end of the mouthpiece element may be left uncovered by the wrapper. The wrapper may be a paper wrapper, such as a cigarette wrapper or tipping paper. The wrapper may be adhered by an adhesive.

The mouthpiece airflow channel may be constructed to have any suitable dimensions to achieve a desired RTD. The plurality of intermediate channels may have a combined cross sectional area of 10 mm² to 20 mm² or from 12 mm² to 16 mm². The discharge portion of the airflow channel may have a cross sectional area of 2 mm² to 5 mm² or from 3 mm² to 4 mm². The mouthpiece element may exhibit an RTD of 75 mmWG or greater, 100 mmWG or greater, or 120 mmWG or greater. The RTD may be 200 mmWG or less, 160 mmWG or less, or 140 mmWG or less. For example, the RTD may be from 75 to 200 mmWG, preferably from 100 to 160 mmWG, more preferably from 120 to 140 mmWG. In one embodiment, the RTD is about 130 mmWG.

The invention is defined in the claims.

Examples will now be further described with reference to the figures in which:
FIGURE 1A is a perspective view of an inhaler article according to an embodiment.
FIGURE 1B is a cross-sectional view of the inhaler article of FIGURE 1A according to an embodiment.
FIGURE 2A is a perspective view of an inhaler article according to an embodiment.
FIGURE 2B is a cross-sectional view of the inhaler article of FIGURE 2A according to an embodiment.
FIGURE 3A is a cross-sectional side view of the inhaler article of FIGURE 1A according to an embodiment.
FIGURE 3B is a cross-sectional side view of the inhaler article of FIGURE 1A coupled with a holder according to an embodiment.
FIGURE 3C is a cross-sectional side view of the inhaler article of FIGURE 1A coupled with a holder and showing an airflow pattern according to an embodiment.
FIGURE 4A is a cross-sectional side view of a mouthpiece element of the inhaler article of FIGURE 1A according to an embodiment.
FIGURE 4B is a perspective view of the mouthpiece element of FIGURE 4A.
FIGURE 5A is a cross-sectional side view of the mouthpiece element of FIGURE 4A showing an airflow pattern according to an embodiment.
FIGURE 5B is a perspective view of the mouthpiece element of FIGURE 4A showing an airflow pattern according to an embodiment.
FIGURE 6A is a cross-sectional side view of the inhaler article of FIGURE 2A according to an embodiment.
FIGURE 6B is a cross-sectional side view of the inhaler article of FIGURE 2A coupled with a holder according to an embodiment.
FIGURE 6C is a cross-sectional side view of the inhaler article of FIGURE 2A coupled with a holder and showing an airflow pattern according to an embodiment.
FIGURE 7A is a cross-sectional side view of a mouthpiece element of the inhaler article of FIGURE 2A according to an embodiment.
FIGURE 7B is a perspective view of the mouthpiece element of FIGURE 7A.
FIGURE 7C is a transverse cross-sectional view of the mouthpiece element of FIGURE 7A according to an embodiment.
FIGURE 8A is a cross-sectional side view of the mouthpiece element of FIGURE 7A showing an airflow pattern according to an embodiment.
FIGURE 8B is a perspective view of the mouthpiece element of FIGURE 7A showing an airflow pattern according to an embodiment.

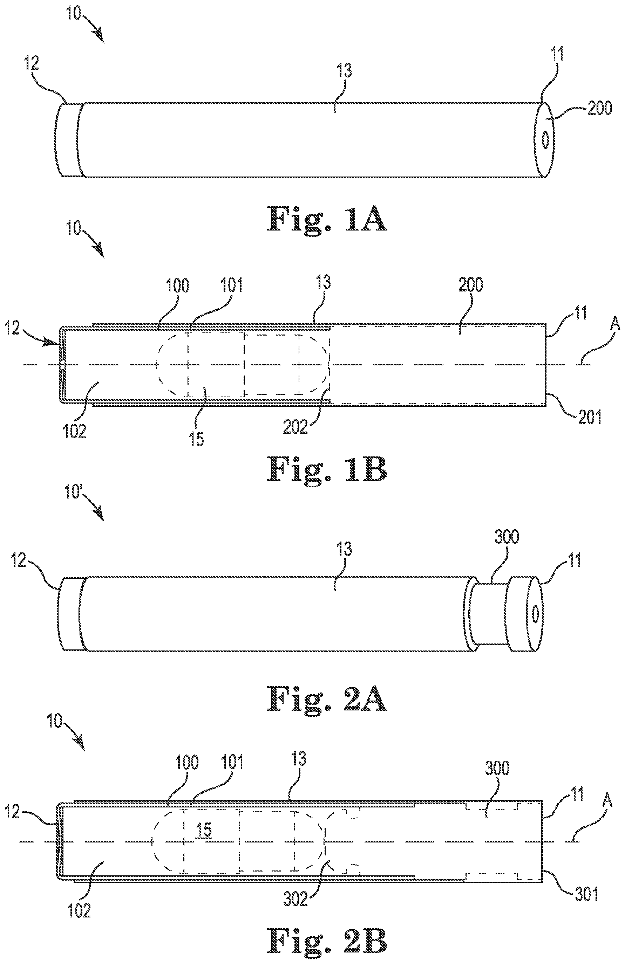

Schematic views of exemplary inhaler articles according to embodiments are shown in FIGURES 1A, 1B, 2A, and 2B. The inhaler article 10, 10' has a first, proximal end 11 and an opposing second, distal end 12. A mouthpiece element 200, 300 is disposed at the proximal end 11 of the inhaler article 10. The outside surface of the inhaler article 10 may be formed, at least in part, by a wrapper 13. The inhaler article 10 comprises an inhaler body 100 with a tubular side wall 101. The inhaler body 100 defines an interior that forms a receptacle 102 for housing an inhalable powder. The inhalable powder may be provided in a capsule 15. The inhaler body 100 further defines a longitudinal center axis A. The mouthpiece element 200, 300 extends along the longitudinal center axis A.

The mouthpiece element 200, 300 is at least partially inserted into the inhaler body 100. The mouthpiece element 200, 300 has a proximal end 201, 301 and a distal end 202, 302. The distal end 202, 302 of the mouthpiece element 200, 300 is received in the interior of the inhaler body 100. As shown in FIGURES 1A and 1B, the entire mouthpiece element 200 may be received in the inhaler body 100. In another embodiment, shown in FIGURES 2A and 2B, the mouthpiece element 300 is only partially inserted inside the inhaler body 100.

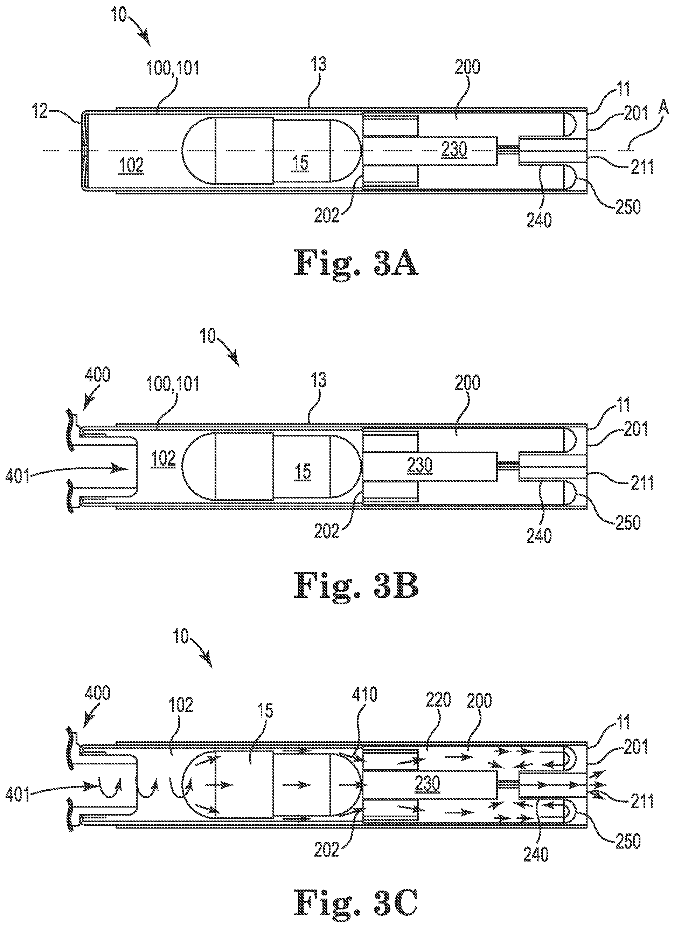

Referring now to FIGURES 3A-3C, cross-sectional views of the inhaler article 10 are shown. As noted above, the inhaler article 10 comprises an inhaler body 100 with a tubular side wall 101. The inhaler body 100 defines an interior that forms a receptacle 102 for housing an inhalable powder. The inhalable powder may be provided in a capsule 15 disposed inside the receptacle 102. The inhaler article 10 may have a closed distal end 12. The distal end 12 may be folded closed, for example by a fan fold. The receptacle 102 may be limited at its proximal end by the distal end 202 of the mouthpiece element 200. The receptacle 102 and the mouthpiece element 200 may be assembled together and wrapped in a wrapper 13. The inhaler article 10 comprises a receptacle 102 containing a capsule 15 containing an inhalable powder, and a mouthpiece element 200, wrapped in a wrapper 13.

The inhaler article 10 may be used with a holder 400. The holder 400 may be configured to open the distal end 12 of the inhaler article 10. The holder 400 may be configured to provide an air inlet 401 for the inhaler article 10. The holder 400 may further be configured to pierce the capsule 15. An airflow pattern 410 flowing through the inhaler article 10 is schematically shown by the arrows in FIGURE 3C. Air flows from the air inlet 401, through the receptacle 102, through the mouthpiece element 200, and out the outlet 211 at the proximal end 201 of the mouthpiece element 200. According to an embodiment, a dry powder may be inhaled from the inhaler article 10 at an airflow rate of 5 mL/min or less, or 2 mL/min or less.

A cross-sectional view and a perspective view of the mouthpiece element 200 are shown in FIGURES 4A and 4B, respectively. The mouthpiece element 200 comprises a tubular side wall 203. The tubular side wall 203 may be cylindrical. The tubular side wall 203 may be substantially straight, extending linearly along the length of the mouthpiece element 200. The tubular side wall 203 may extend from the proximal end 201 of the mouthpiece element 200 to the distal end 202 of the mouthpiece element 200. The mouthpiece element 200 comprises an inlet 212 at the distal end 202 and an outlet 211 at the proximal end 201.

The mouthpiece element comprises a tube 240 extending coaxially with the inhaler body 100. The tube 240 comprises an interior surface 241 having an interior diameter D241. A blocker 230 is disposed at the distal end 202 of the mouthpiece element 200. The blocker 230 may be coaxial with the tube 240. The blocker 230 may be arranged to block airflow through the center of the mouthpiece element 200 at the distal end 202. The blocker 230 may be shaped as a cylinder. The blocker 230 has a diameter D230 that is equal to or greater than the interior diameter D241 of the tube 240. The mouthpiece element 200 may further comprise a plurality of brackets 233 extending from the blocker 230 to the side wall 203. The plurality of brackets 233 may be arranged to maintain the blocker 230 in place inside the mouthpiece element 200. The blocker 230 and the tube 240 may be arranged such that a gap 224 is left between the blocker 230 and the tube 240. The gap 224 has an axial length L224.

The space between the blocker 230 and the side wall 203, the gap 224, and the tube 240 together form an airflow channel 220 through the mouthpiece element 200. The space between the blocker 230 and the side wall 203 forms an off-set portion 221 of the airflow channel. The off-set portion 221 of the airflow channel 220 may be arranged around the blocker 230. The off-set portion 221 of the airflow channel 220 may be formed of a plurality of channels disposed about the blocker 230. The plurality of channels may be symmetrically disposed about the blocker.

The tube 240 forms the discharge portion 223 of the airflow channel 220, terminating at an outlet 211 at the proximal end 201 of the mouthpiece element 200. The gap 224 forms the intermediate portion 222 of the airflow channel, connecting the off-set portion 221 and the discharge portion 223.

The mouthpiece element 200 comprises a collector surface 250 comprising a ring 251. The ring 251 surrounds the tube 240 and is disposed proximal to the blocker 230. The ring 251 may be coaxial with the tube 240. The ring 251 may be coaxial with the blocker 230. The ring 251 defines an inner diameter D251 and an outer diameter D252.

The collector surface 250 may extend from the tube 240 to the side wall 203. The collector surface 250 may have any suitable shape. For example, the collector surface 250 may have a curved cross sectional shape in a cross section taken along the longitudinal center axis A of the mouthpiece element 200, as shown in FIGURE 4A. Preferably, the curved cross sectional shape is concave toward the distal end 202 of the mouthpiece element 200. The collector surface 250 is disposed proximally of the blocker 230. The collector surface 250 may be disposed proximally of the gap 224. The collector surface 250 may be disposed proximal to at least a portion of the tube 240. At least a portion of the tube 240 may extend from the collector surface 250 toward the distal end 202 of the mouthpiece element 200. The portion of the tube 240 extending distally from the collector surface 250 may have a length L240.

The length L224 of the gap 224 may be selected in view of the length L250 between a midpoint of the gap 224 and the proximal end of the collector surface 250. A longer length L250 may be coupled with a longer length L224 of the gap 224. A shorter length L250 may be coupled with a shorter length L224 of the gap 224.

The mouthpiece element 200 may have an outer diameter D200 that is slightly smaller than the inner diameter of the inhaler body 100 such that the mouthpiece element 200 may be completely disposed inside the inhaler body 100. The proximal end 201 of the mouthpiece element 200 may be aligned with a proximal end 11 of the inhaler body 10, as shown in FIGURES 3A-3C.

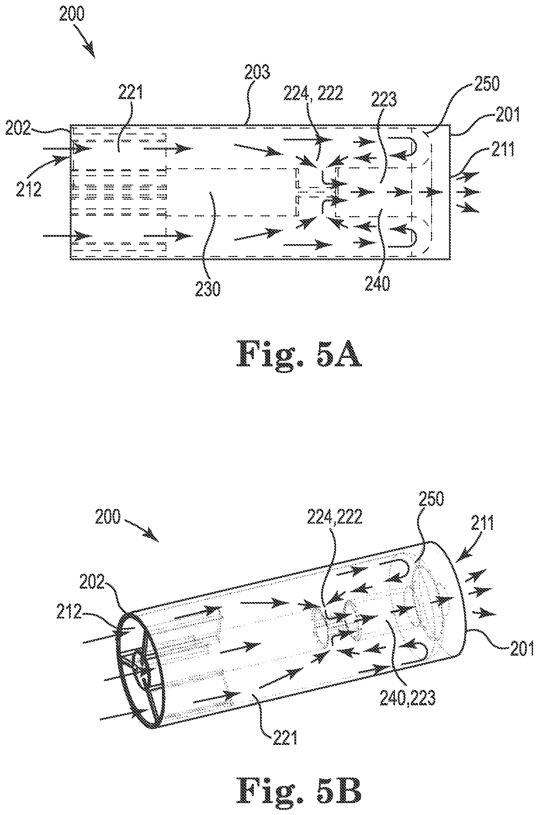

When a user uses the inhaler article 10, air with entrained dry powder particles may flow through the mouthpiece element 200 as shown schematically in FIGURES 5A and 5B. The air and particles may enter the mouthpiece element 200 airflow channel 220 through the inlets 212. The air and particles flow through the off-set portion 221 of the airflow channel 220. Some of the air and particles may flow past the gap 224 toward the collector surface 250 and then turn back toward the gap 224. The air and particles may flow into the gap 224 and through the discharge portion 223. The air and particles may flow out through the outlet 211 at the proximal end of the mouthpiece element 200.

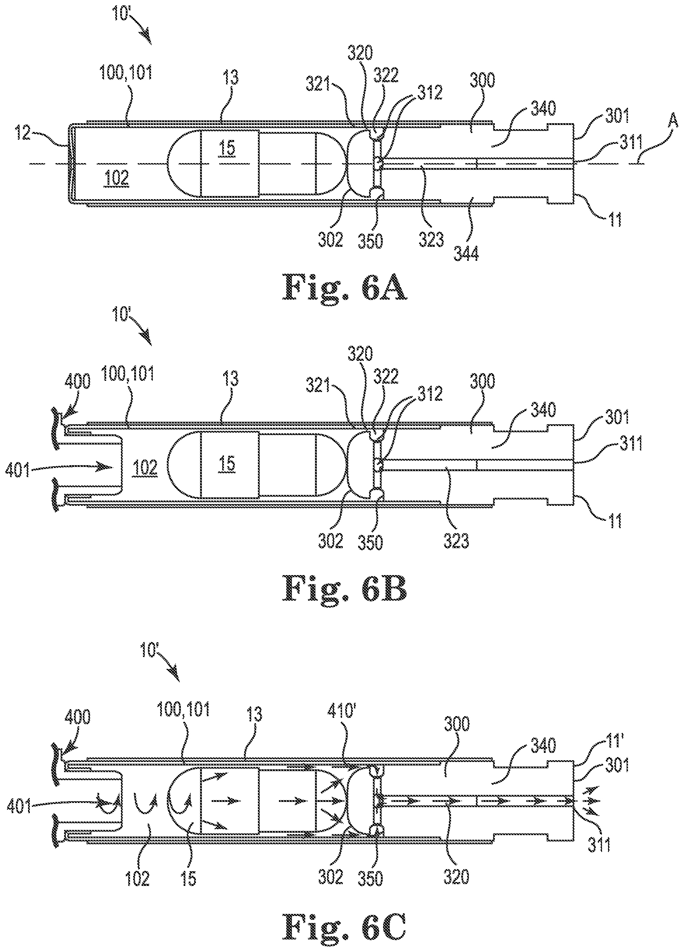

Referring now to FIGURES 6A-6C, cross-sectional views of the inhaler article 10' are shown. As above, the inhaler article 10' comprises an inhaler body 100 with a tubular side wall 101 that defines a longitudinal center axis A. The inhaler body 100 defines an interior that forms a receptacle 102 for housing an inhalable powder. The inhalable powder may be provided in a capsule 15 disposed inside the receptacle. The inhaler article 10' may have a closed distal end 12. The distal end 12 may be folded closed, for example by a fan fold. The receptacle 102 may be limited at its proximal end by the distal end 302 of the mouthpiece element 300.

The inhaler article 10' may be used with a holder 400. The holder 400 may be configured to open the distal end 12 of the inhaler article 10'. The holder 400 may be configured to provide an air inlet 401 for the inhaler article 10'. The holder 400 may further be configured to pierce the capsule 15. An airflow pattern 410' flowing through the inhaler article 10' is schematically shown by the arrows in FIGURE 6C. Air flows from the air inlet 401, through the receptacle 102, through the mouthpiece element 300, and out the outlet 311 at the proximal end 301 of the mouthpiece element 300. According to an embodiment, a dry powder may be inhaled from the inhaler article 10' at an airflow rate of 5 mUmin or less, or 2 mUmin or less.

The inhaler article may further comprise a wrapper 13. The transition from the inhaler body 100 to the mouthpiece element 300 may be covered by the wrapper 13. The wrapper 13 may be wrapped about a portion of the mouthpiece element 300 and at least a portion of the inhaler body 100. For example, the wrapper 13 may extend distally from the main portion 344 of the mouthpiece element 300.

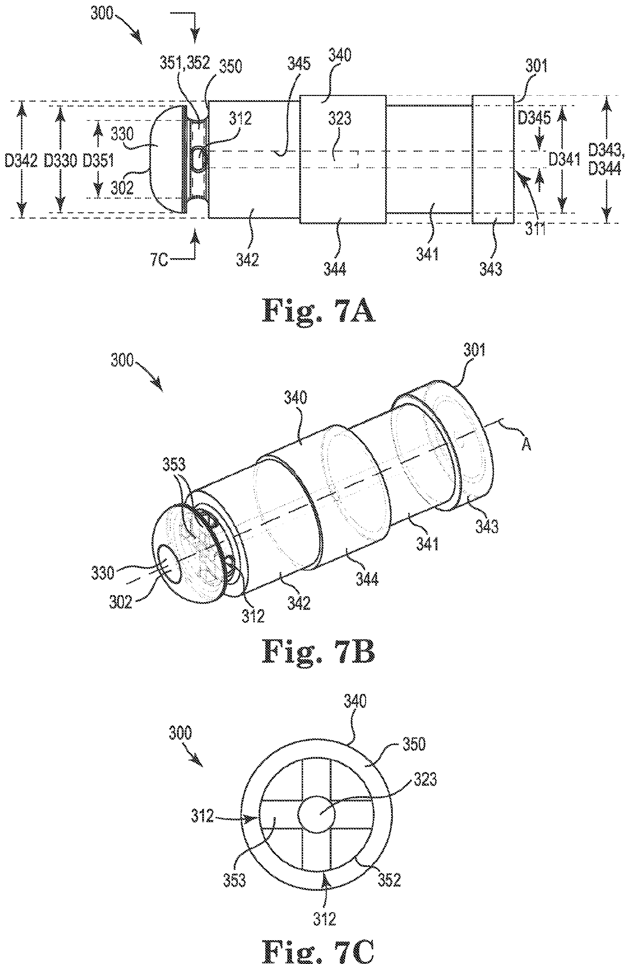

Cross-sectional views and a perspective view of the mouthpiece element 300 are shown in FIGURES 7A-7C. The mouthpiece element 300 extends from the proximal end 301 of the mouthpiece element 300 to the distal end 302 of the mouthpiece element 300. The mouthpiece element 300 comprises an inlet (airflow channel opening) 312 adjacent the distal end 302 and an outlet 311 at the proximal end 301.

The mouthpiece element comprises a tube 340 extending coaxially with the inhaler body 10'. The tube 340 comprises an interior surface 345 having an interior diameter D345. A blocker 330 is disposed at the distal end 302 of the mouthpiece element 300. The blocker 330 may be coaxial with the tube 340. The blocker 330 may be arranged to block airflow through the center of the mouthpiece element 300 at the distal end 302. The blocker 330 has a diameter D330 that is greater than the interior diameter D345 of the tube 340.

The mouthpiece element 300 comprises a recessed portion 351 having a cross-sectional dimension D351 that is smaller than the cross-sectional dimension D330 of the blocker 330. The recessed portion 351 may be formed by a groove circumscribing the mouthpiece element 300. The recessed portion 351 may comprise a plurality of airflow channel openings 312.

A transverse cross-sectional view of the mouthpiece element 300 at the recessed portion 351 is shown in FIGURE 7C. The mouthpiece element 300 may further comprise a plurality of channels 353 extending from the plurality of airflow channel openings 312 to an interior of the tube 340 (to the discharge portion 323 of the airflow channel 320). In a preferred embodiment, the inhaler article comprises four such intermediate channels. The plurality of channels 353 may extend radially inward from the recessed portion 351. The plurality of channels 353 may be orthogonal to the tube 240. The plurality of channels 353 may be distributed symmetrically about the longitudinal center axis A.

The space between the blocker 330 and the tubular side wall 101 of the inhaler body 100, the plurality of channels 353, and the tube 340 together form an airflow channel 320 through the mouthpiece element 300. The space between the blocker 330 and the tubular side wall 101 of the inhaler body 100 forms the off-set portion 321 of the airflow channel. The off-set portion 321 of the airflow channel 320 may be arranged around the blocker 330. The tube 340 forms the discharge portion 323 of the airflow channel 320, terminating in an outlet 311 at the proximal end 301 of the mouthpiece element 300. The plurality of channels 353 form the intermediate portion 322 of the airflow channel, connecting the off-set portion 321 and the discharge portion 323.

The mouthpiece element 300 comprises a collector surface 350 formed by the recessed portion 351. The collector surface 350 may be coaxial with the tube 340. The collector surface 350 may be coaxial with the blocker 330. The collector surface 350 may also be considered to include the recessed wall 352 between the airflow channel openings 312. The recessed portion 351 may form a collector region comprising the collector surface 350, where particles that are not drawn into or do not fall into the discharge portion 323 are collected. The collected particles may later be dislodged by turning the inhaler article upright (with the distal end 12 facing down) and dropped back into the receptacle. Alternatively, the inhaler article and mouthpiece may be cleaned by turning the inhaler article upside down and tapping the inhaler article against a surface (for example, a table). The inhaler article may be tapped several times to cause loose powder to fall out.

The collector surface 350 may extend from the inner diameter D351 to the side wall 101 of the inhaler body 100. The collector surface 350 is disposed proximally of the blocker 330. The collector surface 350 may be disposed distal to the tube 340. The collector surface 350 may have any suitable shape. For example, the collector surface 350 may have a curved cross sectional shape in a cross section taken along the longitudinal center axis A of the mouthpiece element 200, as shown in FIGURE 4A. The curved cross sectional shape may be concave toward the side wall 101 of the inhaler body 100.

The mouthpiece element 300 may comprise multiple sections or portions having varying cross sectional dimensions (for example, diameters). For example, the mouthpiece element 300 (for example, the tube 340) may comprise a first reduced portion 341 separating the mouth end 343 from a main portion 344 of the mouthpiece element 300. The first reduced portion 341 may have a cross sectional dimension D341 (for example, diameter) that is smaller than the cross sectional dimension D343 (for example, diameter) of the mouth end 343 and the cross sectional dimension D344 (for example, diameter) of the main portion 344. The mouthpiece element 300 may further comprise a second reduced portion 342 between the main portion 344 of the mouthpiece element and the recessed portion 351. The second reduced portion 342 may have a cross sectional dimension D342 that is smaller than the cross sectional dimension D344 of the main portion 344 and greater than the cross sectional dimension D351 of the recessed portion 351. The difference in the dimensions of the second reduced portion 342 and the main portion 344 may be only slight. The difference in the dimensions of the second reduced portion 342 and the main portion 344 may account for the thickness of the tubular wall 101 of the inhaler body such that the inhaler wall appears to continue at about the same circumference from the inhaler body 100 to the mouthpiece element 300, as shown in FIGURES 6A-6C.

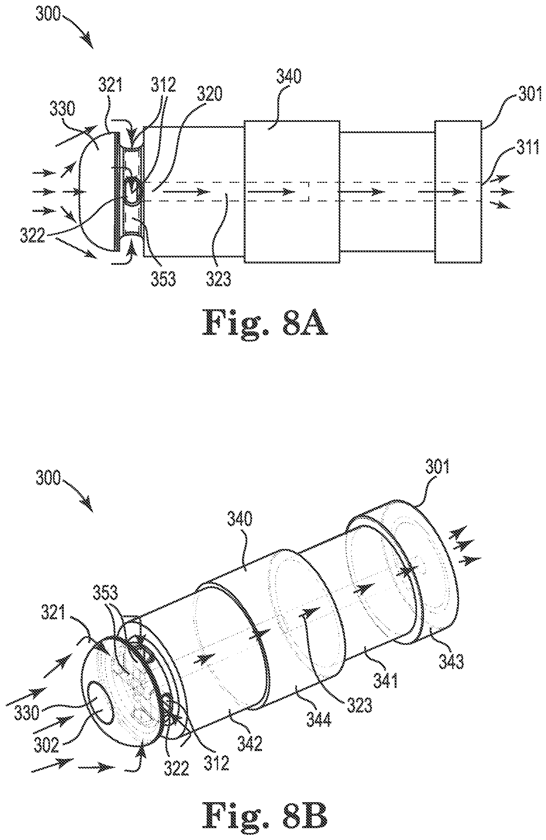

When a user uses the inhaler article 10', air with entrained dry powder particles may flow through the mouthpiece element 300 as shown schematically in FIGURES 8A and 8B. Air and particles are directed by the blocker 330 to flow around the curved top and past the sides of the blocker 330, through the off-set portion 321 of the airflow channel 220. The air and particles enter the mouthpiece element 300 through the airflow channel openings 312. From the airflow channel openings 312, the air and particles may flow into the plurality of channels 353. The air and particles may flow through the plurality of channels 353 (the intermediate portion 322) and continue into the discharge portion 323. The air and particles may flow out through the outlet 311 at the proximal end of the mouthpiece element 300. Any particles that do not enter through the airflow channel openings 312 may be collected by the collector surface 350. For example, loose particles within the receptacle 102 that otherwise could fall out, may be collected on the collector surface when the inhaler article 10' is turned upside down (the proximal end 11 facing downward). The collected particles may later be dislodged by turning the inhaler article upright (with the distal end 12 facing down) and dropped back into the receptacle. Alternatively, the inhaler article and mouthpiece may be cleaned by turning the inhaler article upside down and tapping the inhaler article against a surface (for example, a table). The inhaler article may be tapped several times to cause loose powder to fall out.

## Claims

1. An inhaler article (10; 10') comprising:
an inhaler body (100) comprising a tubular side wall (101) defining an interior and a longitudinal center axis, the interior forming a receptacle (102) for housing an inhalable powder;
a mouthpiece element (200; 300) extending along the longitudinal center axis from a distal end (202; 302) to a proximal end (201; 301), the distal end of the mouthpiece element being received in the interior of the inhaler body, the mouthpiece element comprising:
a tube (240; 340) extending coaxially with the inhaler body and comprising an interior surface (241; 345) having a first diameter (D241; D345);
a blocker (230; 330) disposed at the distal end of the mouthpiece element, coaxial with the tube and having a second diameter (D230; D330) that is greater than the first diameter, the blocker being arranged to block airflow through a center of the mouthpiece element at the distal end;
a collector surface (250; 350) comprising a ring (251) that is coaxial with and disposed proximally of the blocker, the ring defining an inner diameter (D251; D351) and an outer diameter (D252); and
an airflow channel (220; 320) extending through the mouthpiece element and comprising an off-set portion (221; 321) arranged around the blocker, a discharge portion (223; 323) extending to an outlet (211; 311) at the proximal end, and an intermediate portion (222; 322) connecting the off-set portion and the discharge portion.

2. The inhaler article (10; 10') of claim 1, wherein the tube (240; 340) defines an outlet (211; 311) at the proximal end (201; 301) of the mouthpiece element (200; 300).

3. The inhaler article (10; 10') of any one of claims 1 or 2, further comprising a capsule (15) containing inhalable powder disposed in the receptacle.

4. The inhaler article (10; 10') of any one of claims 1 to 3, wherein the collector surface (250; 350) is configured to collect any loose powder within the inhaler body (100) when the inhaler article is turned upside down such that the proximal end (201; 301) of the mouthpiece (200; 300) points downward.

5. The inhaler article (10; 10') of any one of claims 1 to 4, wherein the mouthpiece element (200; 300) comprises a cylindrical side wall extending from the proximal end (201; 301) to the distal end (202; 302) and circumscribing the tube (240; 340).

6. The inhaler article (10; 10') of any one of claims 1 to 5, wherein the blocker (230; 330) comprises a cylinder coaxial with the longitudinal center axis of the mouthpiece element (200; 300).

7. The inhaler article (10; 10') of any one of claims 1 to 6, wherein the collector surface (250; 350) is disposed proximal to at least a portion of the tube (240; 340).

8. The inhaler article (10; 10') of any one of claims 1 to 7, wherein the collector surface (250; 350) extends from the tube (240; 340) to the side wall.

9. The inhaler article (10; 10') of any one of claims 1 to 8, wherein the collector surface (250; 350) comprises a curved cross sectional shape in a cross section taken along the longitudinal center axis of the mouthpiece element.

10. The inhaler article (10; 10') of any one of claims 1 to 9, wherein the curved cross sectional shape is concave toward the distal end (202) of the mouthpiece element (200).

11. The inhaler article (10; 10') of any one of claims 1 to 10, wherein a gap between the blocker (230; 330) and the tube (240; 340) has a distance of 1 mm to 5 mm.

12. The inhaler article (10; 10') of any one of claims 1 to 11, wherein the mouthpiece element (300) comprises a recessed portion (351) having a cross-sectional dimension that is smaller than a distal end portion of the mouthpiece element.

13. The inhaler article (10; 10') of claim 12, wherein the recessed portion (351) comprises a groove circumscribing the mouthpiece element (300).

14. The inhaler article (10; 10') of claim 12 or 13, wherein the recessed portion (351) defines the collector surface (350).

15. The inhaler article (10; 10') of any one of claims 12 to 14, wherein the recessed portion (351) comprises a plurality of airflow channel openings (312) in fluid communication with an interior of the tube (340).

## Patentansprüche

1. Inhalatorartikel (10; 10'), umfassend:
einen Inhalatorkörper (100), umfassend eine rohrförmige Seitenwand (101), die ein Inneres und eine Längsmittelachse definiert, wobei das Innere eine Aufnahme (102) zum Aufnehmen eines inhalierbaren Pulvers bildet;
ein Mundstückelement (200; 300), das sich entlang der Längsmittelachse von einem distalen Ende (202; 302) zu einem proximalen Ende (201; 301) erstreckt, wobei das distale Ende des Mundstückelements in dem Inneren des Inhalatorkörpers aufgenommen wird, wobei das Mundstückelement umfasst:
ein Rohr (240; 340), das sich koaxial zu dem Inhalatorkörper erstreckt und eine Innenfläche (241; 345) umfasst, die einen ersten Durchmesser (D241; D345) aufweist;
einen Blocker (230; 330), der an dem distalen Ende des Mundstückelements angeordnet ist, koaxial mit dem Rohr verläuft und einen zweiten Durchmesser (D230; D330) aufweist, der größer ist als der erste Durchmesser, wobei der Blocker zum Blockieren des Luftstroms durch eine Mitte des Mundstückelements an dem distalen Ende angeordnet ist;
eine Sammelfläche (250; 350), umfassend einen Ring (251), der koaxial zu dem und proximal des Blockers angeordnet ist, wobei der Ring einen Innendurchmesser (D251; D351) und einen Außendurchmesser (D252) definiert; und
einen Luftstromkanal (220; 320), der sich durch das Mundstückelement erstreckt und einen versetzten Abschnitt (221; 321), der um den Blocker herum angeordnet ist, einen Auslassabschnitt (223; 323), der sich zu einem Auslass (211; 311) an dem proximalen Ende erstreckt, und einen Zwischenabschnitt (222; 322), der den versetzten Abschnitt und den Auslassabschnitt verbindet, umfasst.

2. Inhalatorartikel (10; 10') nach Anspruch 1, wobei das Rohr (240; 340) einen Auslass (211; 311) an dem proximalen Ende (201; 301) des Mundstückelements (200; 300) definiert.

3. Inhalatorartikel (10; 10') nach einem beliebigen der Ansprüche 1 oder 2, ferner umfassend eine ein inhalierbares Pulver enthaltende Kapsel (15), die in der Aufnahme angeordnet ist.

4. Inhalatorartikel (10; 10') nach einem beliebigen der Ansprüche 1 bis 3, wobei die Sammelfläche (250; 350) zum Sammeln von losem Pulver innerhalb des Inhalatorkörpers (100) ausgelegt ist, wenn der Inhalatorartikel umgedreht wird, sodass das proximale Ende (201; 301) des Mundstücks (200; 300) nach unten zeigt.

5. Inhalatorartikel (10; 10') nach einem beliebigen der Ansprüche 1 bis 4, wobei das Mundstückelement (200; 300) eine zylindrische Seitenwand umfasst, die sich von dem proximalen Ende (201; 301) zu dem distalen Ende (202; 302) erstreckt und das Rohr (240; 340) umhüllt.

6. Inhalatorartikel (10; 10') nach einem beliebigen der Ansprüche 1 bis 5, wobei der Blocker (230; 330) einen Zylinder umfasst, der koaxial zu der Längsmittelachse des Mundstückelements (200; 300) ist.

7. Inhalatorartikel (10; 10') nach einem beliebigen der Ansprüche 1 bis 6, wobei die Sammelfläche (250; 350) proximal zu wenigstens einem Abschnitt des Rohrs (240; 340) angeordnet ist.

8. Inhalatorartikel (10; 10') nach einem der Ansprüche 1 bis 7, wobei sich die Sammelfläche (250; 350) von dem Rohr (240; 340) zu der Seitenwand erstreckt.

9. Inhalatorartikel (10; 10') nach einem beliebigen der Ansprüche 1 bis 8, wobei die Sammelfläche (250; 350) eine gekrümmte Querschnittsform in einem entlang der Längsmittelachse des Mundstückelements genommenen Querschnitt aufweist.

10. Inhalatorartikel (10; 10') nach einem beliebigen der Ansprüche 1 bis 9, wobei die gekrümmte Querschnittsform in Richtung des distalen Endes (202) des Mundstückelements (200) konkav ist.

11. Inhalatorartikel (10; 10') nach einem beliebigen der Ansprüche 1 bis 10, wobei ein Spalt zwischen dem Blocker (230; 330) und dem Rohr (240; 340) einen Abstand von 1 mm bis 5 mm aufweist.

12. Inhalatorartikel (10; 10') nach einem beliebigen der Ansprüche 1 bis 11, wobei das Mundstückelement (300) einen vertieften Abschnitt (351) mit einer Querschnittsabmessung umfasst, die kleiner ist als ein distaler Endabschnitt des Mundstückelements.

13. Inhalatorartikel (10; 10') nach Anspruch 12, wobei der vertiefte Abschnitt (351) eine das Mundstückelement (300) umhüllende Nut aufweist.

14. Inhalatorartikel (10; 10') nach Anspruch 12 oder 13, wobei der vertiefte Abschnitt (351) die Sammelfläche (350) definiert.

15. Inhalatorartikel (10; 10') nach einem der Ansprüche 12 bis 14, wobei der vertiefte Abschnitt (351) eine Vielzahl von Öffnungen (312) des Luftstromkanals in Fluidverbindung mit einem Inneren des Rohrs (340) aufweist.

## Revendications

1. Article inhalateur (10 ; 10') comprenant :
un corps d'inhalateur (100) comprenant une paroi latérale tubulaire (101) définissant un intérieur et un axe central longitudinal, l'intérieur formant un réceptacle (102) destiné à loger une poudre inhalable ;
un élément d'embout buccal (200 ; 300) s'étendant le long de l'axe central longitudinal d'une extrémité distale (202 ; 302) à une extrémité proximale (201 ; 301), l'extrémité distale de l'élément d'embout buccal étant reçue à l'intérieur du corps d'inhalateur, l'élément d'embout buccal comprenant :
un tube (240 ; 340) s'étendant coaxialement au corps d'inhalateur et comprenant une surface intérieure (241 ; 345) ayant un premier diamètre (D241 ; D345) ;
un bloqueur (230 ; 330) disposé à l'extrémité distale de l'élément d'embout buccal, coaxial au tube et ayant un deuxième diamètre (D230 ; D330) qui est plus grand que le premier diamètre, le bloqueur étant agencé pour bloquer un écoulement d'air à travers un centre de l'élément d'embout buccal à l'extrémité distale ;
une surface collectrice (250 ; 350) comprenant une bague (251) qui est coaxiale au bloqueur et disposée de manière proximale par rapport à celui-ci, la bague définissant un diamètre intérieur (D251 ; D351) et un diamètre extérieur (D252) ; et
un conduit d'écoulement d'air (220 ; 320) s'étendant à travers l'élément d'embout buccal et comprenant une portion décalée (221 ; 321) agencée autour du bloqueur, une portion de décharge (223 ; 323) s'étendant jusqu'à une sortie (211 ; 311) à l'extrémité proximale, et une portion intermédiaire (222 ; 322) raccordant la portion décalée et la portion de décharge.

2. Article inhalateur (10 ; 10') selon la revendication 1, dans lequel le tube (240 ; 340) définit une sortie (211 ; 311) à l'extrémité proximale (201 ; 301) de l'élément d'embout buccal (200 ; 300).

3. Article inhalateur (10 ; 10') selon l'une quelconque des revendications 1 ou 2, comprenant en outre une capsule (15) contenant de la poudre inhalable disposée dans le réceptacle.

4. Article inhalateur (10 ; 10') selon l'une quelconque des revendications 1 à 3, dans lequel la surface collectrice (250 ; 350) est configurée pour collecter toute poudre libre au sein du corps d'inhalateur (100) lorsque l'article inhalateur est retourné tête en bas de telle sorte que l'extrémité proximale (201 ; 301) de l'embout buccal (200 ; 300) pointe vers le bas.

5. Article inhalateur (10 ; 10') selon l'une quelconque des revendications 1 à 4, dans lequel l'élément d'embout buccal (200 ; 300) comprend une paroi latérale cylindrique s'étendant de l'extrémité proximale (201 ; 301) à l'extrémité distale (202 ; 302) et entourant le tube (240 ; 340).

6. Article inhalateur (10 ; 10') selon l'une quelconque des revendications 1 à 5, dans lequel le bloqueur (230 ; 330) comprend un cylindre coaxial à l'axe central longitudinal de l'élément d'embout buccal (200 ; 300).

7. Article inhalateur (10 ; 10') selon l'une quelconque des revendications 1 à 6, dans lequel la surface collectrice (250 ; 350) est disposée proximale à au moins une portion du tube (240 ; 340) .

8. Article inhalateur (10 ; 10') selon l'une quelconque des revendications 1 à 7, dans lequel la surface collectrice (250 ; 350) s'étend du tube (240 ; 340) à la paroi latérale.

9. Article inhalateur (10 ; 10') selon l'une quelconque des revendications 1 à 8, dans lequel la surface collectrice (250 ; 350) comprend une forme de coupe transversale courbée dans une coupe transversale prise le long de l'axe central longitudinal de l'élément d'embout buccal.

10. Article inhalateur (10 ; 10') selon l'une quelconque des revendications 1 à 9, dans lequel la forme de coupe transversale courbée est concave vers l'extrémité distale (202) de l'élément d'embout buccal (200).

11. Article inhalateur (10 ; 10') selon l'une quelconque des revendications 1 à 10, dans lequel un écartement entre le bloqueur (230 ; 330) et le tube (240 ; 340) a une distance de 1 mm à 5 mm.

12. Article inhalateur (10 ; 10') selon l'une quelconque des revendications 1 à 11, dans lequel l'élément d'embout buccal (300) comprend une portion évidée (351) ayant une dimension de coupe transversale qui est plus petite qu'une portion d'extrémité distale de l'élément d'embout buccal.

13. Article inhalateur (10 ; 10') selon la revendication 12, dans lequel la portion évidée (351) comprend une rainure entourant l'élément d'embout buccal (300).

14. Article inhalateur (10 ; 10') selon la revendication 12 ou 13, dans lequel la portion évidée (351) définit la surface collectrice (350).

15. Article inhalateur (10 ; 10') selon l'une quelconque des revendications 12 à 14, dans lequel la portion évidée (351) comprend une pluralité d'ouvertures de conduit d'écoulement d'air (312) en communication fluidique avec un intérieur du tube (340).
